# EUROPEAN PATENT APPLICATION

(11) **EP 1 741 898 A2**
(43) Date of publication of application: **10.01.2007**
(21) Application number: 06253479.7
(22) Date of filing: 03.07.2006
(51) Int. Cl.: F02C 3/28, F02C 3/22, F01K 23/06

(54) **Syngas turbine**

(30) Priority: 05.07.2005 US 174767
(71) Applicant: The General Electric Company, Schenectady NY 12345 (US)
(72) Inventor: Dean, Anthony John, Scotia New York 12302 (US); Ali, Mohamed Ahmed, Clifton Park New York 12065 (US)
(74) Representative: Bedford, Grant Richard

(57) **Abstract**

A syngas turbine (10) comprises a combustor (12) for reacting a hydrocarbon fuel (14) with at least one of an oxidant (16) and steam (18) at an elevated temperature and pressure to produce a synthesis gas (20) enriched with carbon monoxide and hydrogen gas. A turbo-expander (22) is in flow communication with the combustor (12) for extracting work from and for quenching the synthesis gas (20).

## Description

The invention relates generally to generating synthesis gas and more specifically to integrating a syngas turbine into a synthesis gas generation system.

Currently complex industrial plants are built around the globe to produce synthesis gases for use in a variety of applications including conversion of natural gas to useful liquid fuels, generation of hydrogen-enriched gases and other processes. One challenge faced by these complex industrial plants is the capital expenditure that is required to build these plants. In addition, most of these plants involve a variety of chemical processing stages that are independently developed from one another leading to poor thermodynamic and physical integration between stages.

Conversion efficiency of natural gas to liquid product is another important technical and economic driver. A technical requirement is to reduce the temperature of the syngas at the exit of the synthesis gas generator prior to further processing. This temperature reduction, termed quenching, provides the opportunity to recycle heat of energy to improve overall conversion efficiency.

Accordingly, there is a need to recycle the energy available from the quench process as efficiently as possible. Accordingly, there is a need to improve gas synthesis processing by lowering the complexity of the systems through innovative approaches and improved integration. Improving the overall complexity of these systems will drastically reduce the capital expenditure needed to build these plants.

According to one aspect, the present invention provides a syngas turbine comprises a combustor for reacting a hydrocarbon fuel with at least one of an oxidant and steam at an elevated temperature and pressure to produce a synthesis gas enriched with carbon monoxide and hydrogen gas. A turbo-expander is in flow communication with the combustor for extracting work from and for quenching the synthesis gas.

Various features, aspects, and advantages of the present invention will become better understood when the following detailed description is read with reference to the accompanying drawings in which like characters represent like parts throughout the drawings, wherein:
FIG. 1 is a schematic representation of one embodiment of the instant invention.
Fig. 2 is a schematic of a representative gas to liquid system.
Fig. 3 is a schematic representation of another embodiment of the instant invention.
Fig. 4 is a schematic representation of another embodiment of the instant invention.
Fig. 5 is a schematic representation of another embodiment of the instant invention.
Fig. 6 is a schematic representation of another embodiment of the instant invention.
Fig. 7 is a schematic representation of another embodiment of the instant invention.
Fig. 8 is a schematic representation of another embodiment of the instant invention.
Fig. 9 is a schematic representation of another embodiment of the instant invention.
Fig. 9a is a cross-sectional view of the combustor of Fig. 9.
Fig. 10 is a schematic representation of another embodiment of the instant invention.
Fig. 10a is a cross-sectional view of the combustor of Fig. 10.
Fig. 11 is a schematic representation of another embodiment of the instant invention.

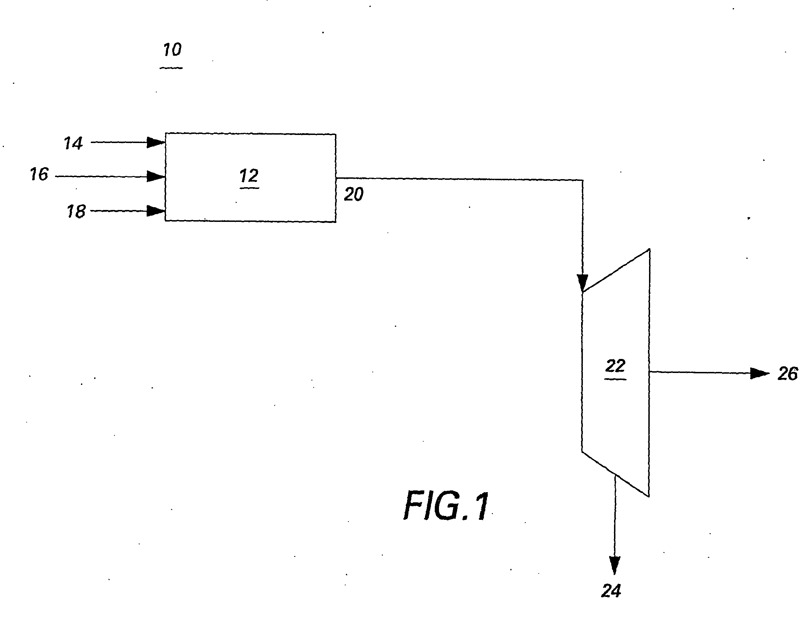

A syngas turbine 10 comprises a combustor 12 for reacting a hydrocarbon fuel 14, for example natural gas, with at least one of an oxidant 16, for example oxygen, and steam 18 at an elevated temperature and pressure to produce a synthesis gas 20 enriched with carbon monoxide (CO) and hydrogen gas (H₂), as shown in FIG. 1. Syngas turbine 10 further comprises a turbo-expander 22 in flow communication with the combustor 12 for extracting work 24 from and for quenching the synthesis gas 20 to create a quenched syngas 26. In one embodiment, the turbo-expander 22 is a multi-stage axial flow turbine. In another embodiment, turbo-expander 22 or combustor 12 are cooled by steam or by nitrogen.

Conventional synthesis gas generation is commonly used in a variety of applications. One process that requires generation of synthesis gas is the gas-to-liquid (GTL) process. A significant fraction of the world's natural gas reserves are too far from large markets to be transported by pipeline. One technology that is being investigated is the GTL process. In the GTL process natural gas is converted into a liquid hydrocarbon, for example, liquid diesel. Once converted into a liquid, the fuel becomes easy to store and transport.

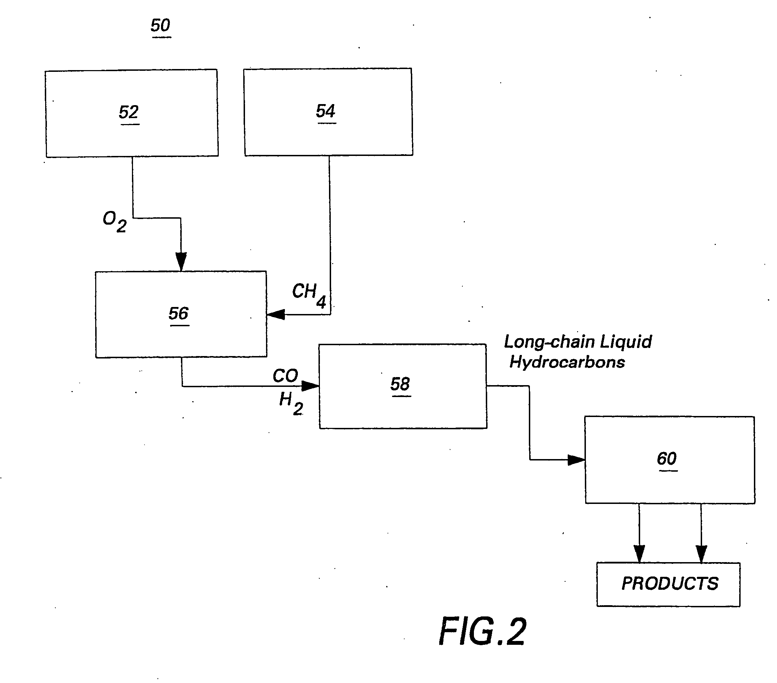

A conventional GTL system 50 includes an air separation unit 52, a gas processing unit 54, a gas synthesis unit 56, a Fischer-Tropsch processing unit 58, and a cracking unit 60, as shown in FIG. 2.

Air separation unit 52 separates air into nitrogen (N₂), oxygen (O₂) and other gases and the gas-processing unit 54 prepares raw natural gas for conversion in the gas synthesis unit 56 by filtering and reducing the levels of impurities such as sulfur. The oxygen from the air separation unit 52 and the natural gas from the gas-processing unit 54 are directed to the gas synthesis unit 56, where the oxygen reacts with the natural gas under fuel-rich conditions to form hydrogen gas (H₂) and carbon monoxide (CO).

Next, the hydrogen gas and the carbon monoxide are introduced into the Fischer-Tropsh processing unit 58 and, through catalysis, are recombined into long-chain liquid hydrocarbons. Finally, in a cracking unit 60, the liquid hydrocarbons are converted and fractionated into products that can be used immediately, are readily transportable and can be blended easily to form products such as synthetic diesel fuel, synthetic kerosene, ethanol, dimethyl ether, naphtha or combinations or derivates thereof.

As discussed above, one issue with conventional GTL systems is that they are complex, inefficient and have an extremely large footprint. The following embodiments of the invention discuss systems that can lower the overall complexity of syngas generation processes; improve the operating efficiencies of these processes; and/or provide a much simpler and smaller overall footprint for syngas production systems, especially for GTL systems. These modifications enhance the attractiveness of syngas processing, especially GTL processing.

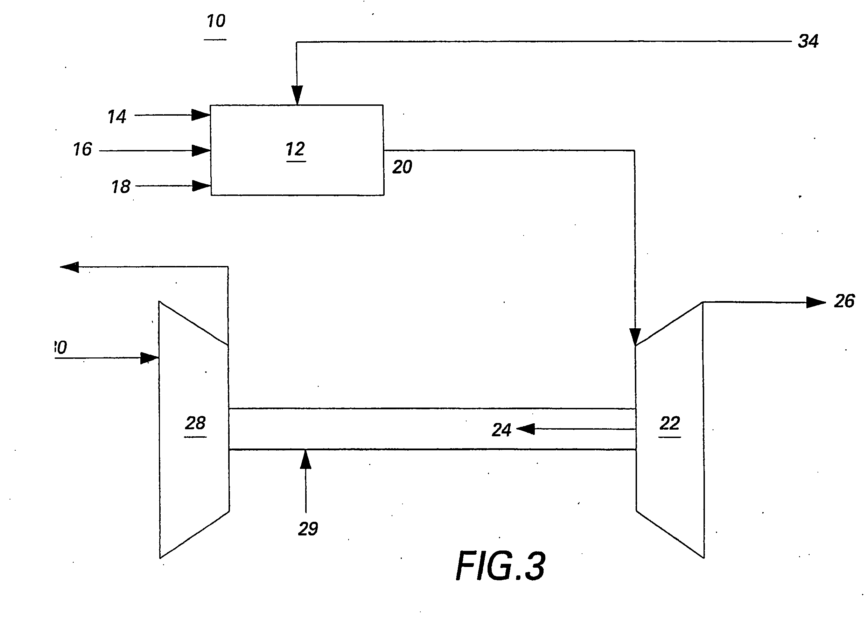

A syngas turbine 10 comprises a combustor 12 for reacting a hydrocarbon fuel 14, for example natural gas, with at least one of an oxidant 16 and steam 18 at an elevated temperature and pressure to produce a synthesis gas 20 enriched with carbon monoxide (CO) and hydrogen gas (H₂), as shown in FIG. 3. Syngas turbine 10 further comprises a turbo-expander 22 in flow communication with the combustor 12 for extracting work 24 from and for quenching the synthesis gas 20 to create a quenched synthesis gas 26. As discussed above, a technical requirement related to syngas is that the temperature of the generated syngas must be reduced, for example from about 2000°F to about 500°F, prior to further processing. Conventionally, synthesis gas is quenched by removing heat in a boiler that produces steam by boiling water. In addition, water or steam can be introduced into the syngas flow to lower the temperature. Conventional synthesis gas generators have a relatively long residence time and products include solid carbon. In order to remove the solid carbon, the syngas is bubbled through water prior to further processing. In contrast, in syngas turbine 10 the exit temperature of the syngas is defined by the inlet temperature, the turbine pressure ratio, the turbine efficiency and the cooling or dilution used, leading to an efficient and effective quench process. Additionally, because of the short residence times, solid carbon formation is minimized.

In one embodiment, turbo-expander 22 is coupled to an air compressor 28. The work 24 generated by turbo-expander 22 is mechanically transferred, for example through a shaft 29, to air compressor 28. The air compressor 28 compresses the air flowing into it via inlet 30 and transmits compressed air out through outlet 32. The compressed air can be utilized in a variety of fashions including as a feed stream into an air separation unit (not shown) to generate the oxidant 16 provided into combustor 12. As shown in FIG. 3, the inlet streams (14, 16, 18) provided into combustor 12 may further include a recycled tail-gas 34, which tail-gas 34 contains fuel-bearing gas that is recycled from a down-stream source. Typically, although not necessarily, in a GTL application, the tail-gas is the gas phase product from the Fischer-Tropsh processing unit. A very large Fischer-Tropsh processing unit may consume all of the reactant, but practical systems limit the size of the reactor. Accordingly, there is typically a portion of reactants available for recycle.

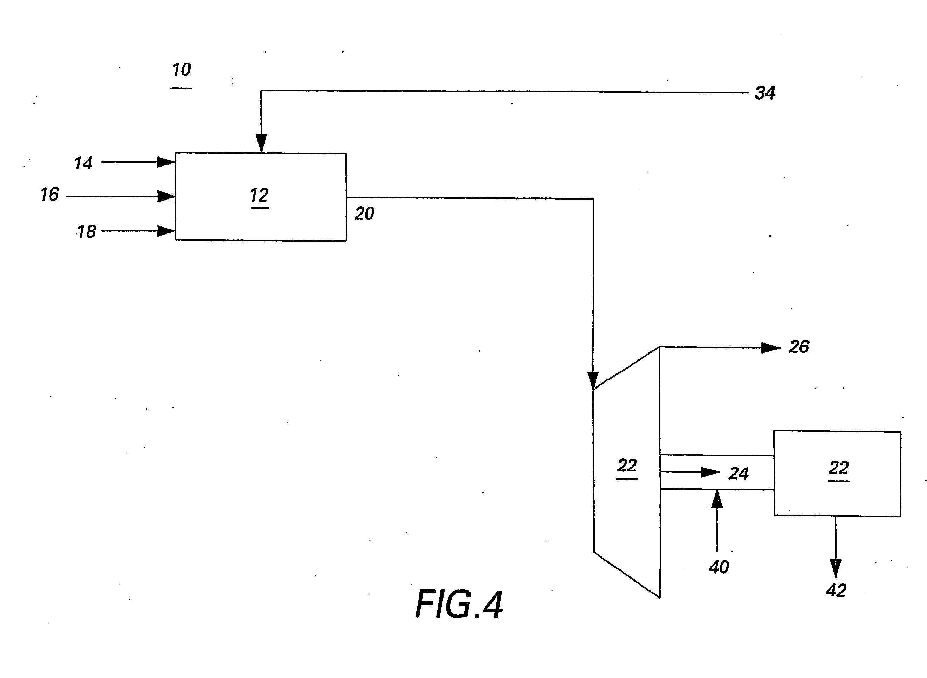

A syngas turbine 10 comprises a combustor 12 for reacting a hydrocarbon fuel 14 with at least one of an oxidant 16 and steam 18 at an elevated temperature and pressure to produce a synthesis gas 20 enriched with carbon monoxide (CO) and hydrogen gas (H₂), as shown in FIG. 4. Syngas turbine 10 further comprises a turbo-expander 22 in flow communication with the combustor 12 for extracting work 24 from and for quenching the synthesis gas 20. In one embodiment, turbo-expander 22 is coupled to a generator 36, typically via a shaft 40. The work 24 generated by turbo-expander 22 is mechanically transferred through shaft 40 to generator 36. The generator 36 converts the mechanical energy provided through shaft 40 into electricity 42. The generated electricity 42 can be utilized in a variety of fashions. As shown in Figure 4, the inlet streams provided into combustor 12 may further include a recycled tail-gas 34, which tail-gas 34 contains fuel-bearing gas that is recycled from a down-stream source.

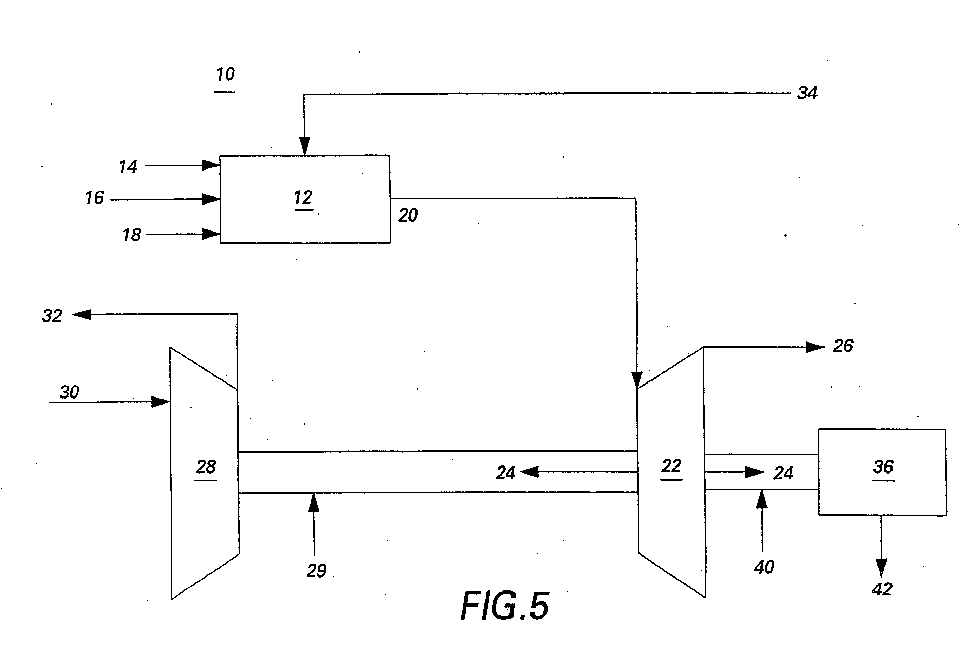

A syngas turbine 10 comprises a combustor 12 for reacting a hydrocarbon fuel 14 with at least one of an oxidant 16 and steam 18 at an elevated temperature and pressure to produce a synthesis gas 20 enriched with carbon monoxide (CO) and hydrogen gas (H₂), as shown in FIG. 5. Syngas turbine 10 further comprises a turbo-expander 22 in flow communication with the combustor 12 for extracting work 24 from and for quenching the synthesis gas 20. In one embodiment, turbo-expander 22 is coupled to generator 36, typically via shaft 40 and to air compressor 28, typically via shaft 29. The work 24 generated by turbo-expander 22 is mechanically transferred through shaft 40, to generator 36 and through shaft 29 to air compressor 28. The generator 36 converts the mechanical energy provided through shaft 40 into electricity 42. The generated electricity 42 can be utilized in a variety of fashions. The air compressor 28 compresses the air stream flowing into it via inlet 30 and transmits compressed air out through outlet 32. The compressed air can be utilized in a variety of fashions including as a feed into an air separation unit (not shown) to generate the oxidant 16 provided into combustor 12. The work 24 extracted from turbo-expander 22 can be dedicated to either shaft 29 or to shaft 40, or alternatively, the work 24 can be shared between shafts 29 and 40. In one embodiment, shafts 29 and shaft 40 are combined into a single shaft that mechanically transfers the work 24 generated by turbo-expander 22 to both the air compressor 28 and to the generator 36.

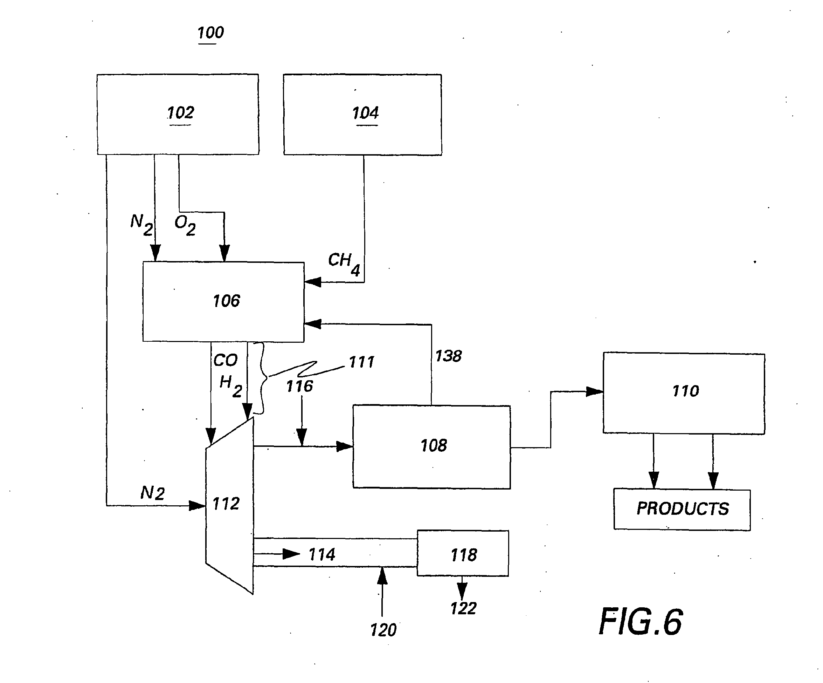

A GTL system 100 includes an air separation unit 102, a gas processing unit 104, a combustor 106, a Fischer-Tropsch processing unit 108, a cracking unit 110, and a turbo-expander 112, as shown in FIG. 6.

Air separation unit 102 separates air into nitrogen (N₂), oxygen (O₂) and other gases and the gas-processing unit 104 prepares natural gas for conversion in the combustor 106. The oxygen from the air separation unit 102 and the natural gas from the gas-processing unit 104 are directed to the combustor 106, where the natural gas and the oxygen are reacted at an elevated temperature and pressure to produce a synthesis gas 111 that is enriched with CO and H₂. The synthesis gas 111 is directed into turbo-expander 112 in flow communication with the combustor 106 for extracting work 114 from and for quenching the synthesis gas to produce a quenched synthesis gas 116.

Next, the quenched synthesis gas 116 enriched with hydrogen gas and carbon monoxide is introduced into the Fischer-Tropsh processing unit 108 where through catalysis, the hydrogen gas and the carbon monoxide are recombined into long-chain liquid hydrocarbons. Finally in the cracking unit 110, the liquid hydrocarbons are converted and fractionated into products that can be used immediately, are readily transportable and can be used to form products such as synthetic diesel fuel, synthetic kerosene, ethanol, dimethyl ether, naphtha, and combinations or derivates thereof.

In one embodiment, turbo-expander 112 is coupled to a generator 118, typically via a shaft 120. The work 114 generated by turbo-expander 112 is mechanically transferred through shaft 120 to generator 118. The generator 118 converts the mechanical energy provided through shaft 120 into electricity 122. The generated electricity 122 can be utilized in a variety of fashions. In one embodiment, the turbo-expander 112 is a multi-stage axial flow turbine. In another embodiment, turbo-expander 112 or combustor 106 are cooled by steam or by nitrogen. In yet another embodiment, turbo-expander 112 or combustor 106 are cooled by nitrogen (N₂) generated by air separation unit 102. The inlet streams provided into combustor 106 may further include a recycled tail-gas 138, which tail-gas 138 contains fuel-bearing gas that is recycled from a down-stream source. Typically, although not necessarily, the tail-gas 138 is a gas phase product from the Fischer-Tropsh processing unit 108.

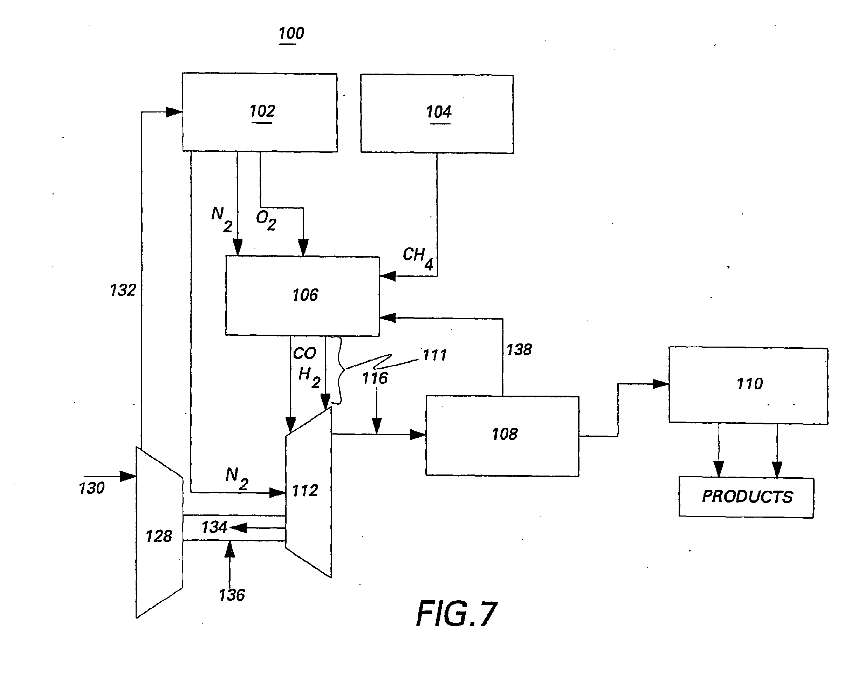

FIG. 7 depicts a GTL system that is the same as the GTL system discussed with respect to FIG. 6, except that it further comprises an air compressor 128 coupled to turbo-expander 112. The work 134 generated by turbo-expander 112 is mechanically transferred, for example through a shaft 136, to air compressor 128. The air compressor 128 compresses the air flowing into it via inlet 130 and transmits compressed air out through outlet 132. The compressed air can be utilized in a variety of fashions including as a feed stream into air separation unit 102 to generate the oxidant provided into combustor 106. The inlet streams provided into combustor 106 may further include a recycled tail-gas 138, which tail-gas 138 contains fuel-bearing gas that is recycled from a down-stream source. Typically, although not necessarily, the tail-gas 138 is a gas phase product from the Fischer-Tropsh processing unit 108.

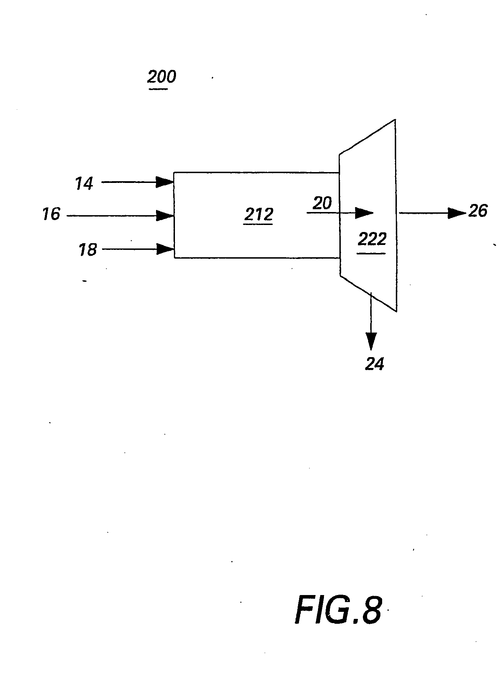

In accordance with another embodiment of the invention, FIGS. 8-11 depict a series of close-coupled syngas turbines that can be substituted into each of the systems described with reference to FIGS. 1-7. These close-coupled syngas turbine configurations, as discussed below, enable a much smaller physical footprint in a syngas generation processing facility, for example a GTL processing facility; the close-coupled syngas turbine configurations are simpler designs and require less piping, cooling, coupling and sealing between components, thereby lowering the overall complexity of the systems; and because of the close proximity between the combustor and the turbine, the pressure loss between these components is significantly reduced, thereby enhancing the overall process efficiency.

A close-coupled syngas turbine 200 comprises a combustor 212 for reacting a hydrocarbon fuel 14 with at least one of an oxidant 16 and steam 18 at an elevated temperature and pressure to produce a synthesis gas 20 enriched with carbon monoxide (CO) and hydrogen gas (H₂), as shown in FIG. 8. Syngas turbine 200 further comprises a turbo-expander 222 directly coupled to and in flow communication with the combustor 212 for extracting work 24 from and for quenching the synthesis gas 20 to produce a quenched synthesis gas 26.

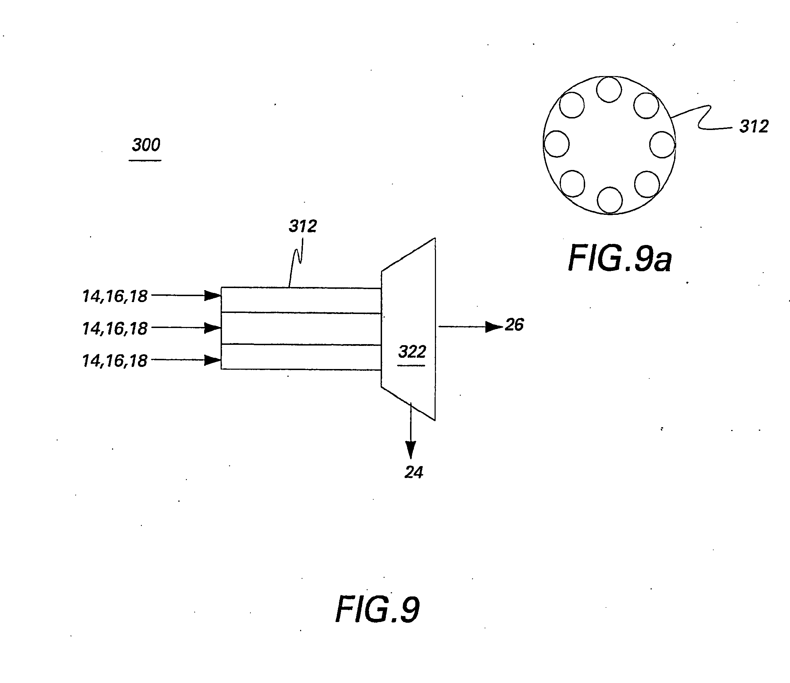

A close-coupled syngas turbine 300 comprises a can-annular combustor 312 for reacting a hydrocarbon fuel 14 with at least one of an oxidant 16 and steam 18 at an elevated temperature and pressure to produce a synthesis gas 20 enriched with carbon monoxide (CO) and hydrogen gas (H₂), as shown in FIG. 9. Syngas turbine 300 further comprises a turbo-expander 322 directly coupled to and in flow communication with the can-annular combustor 312 for extracting work 24 from and for quenching the synthesis gas 20 to produce a quenched synthesis gas 26.

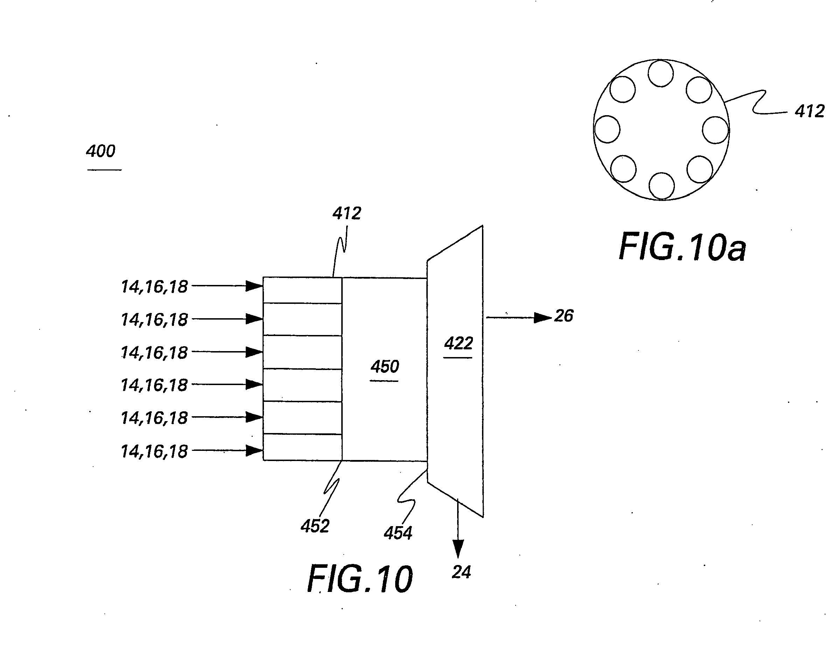

A close-coupled syngas turbine 400 comprises a can-annular combustor 412 for reacting a hydrocarbon fuel 14 with at least one of an oxidant 16 and steam 18 at an elevated temperature and pressure to produce a synthesis gas 20 enriched with carbon monoxide (CO) and hydrogen gas (H₂), as shown in FIG. 10. Syngas turbine 400 further comprises a turbo-expander 422 in flow communication with the can-annular combustor 412 for extracting work 24 from and for quenching the synthesis gas 20 to produce a quenched synthesis gas 26. A transitional piece 450 is directly coupled to the can-annular combustor on a first end 452 and directly coupled to the turbo-expander 422 at the opposite end 454.

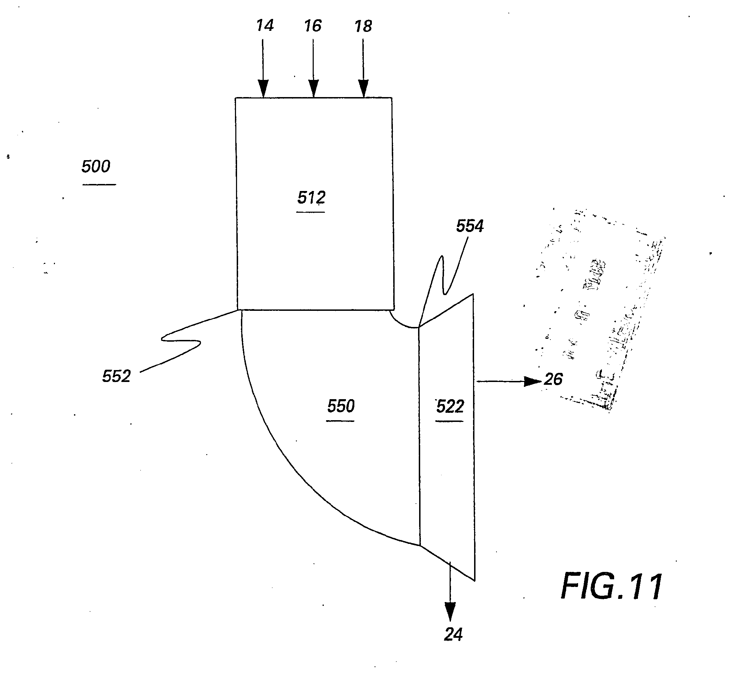

A close-coupled syngas turbine 500 comprises a combustor 512 for reacting a hydrocarbon fuel 14 with at least one of an oxidant 16 and steam 18 at an elevated temperature and pressure to produce a synthesis gas 20 enriched with carbon monoxide (CO) and hydrogen gas (H₂), as shown in FIG. 11. Syngas turbine 500 further comprises a turbo-expander 522 in flow communication with combustor 512 for extracting work 24 from and for quenching the synthesis gas 20 to produce a quenched synthesis gas 26. A transitional piece 550 is directly coupled to the combustor 512 on a first end 552 and directly coupled to the turbo-expander 522 at an opposite end 554.

While only certain features of various aspects and embodiments of the invention have been illustrated and described herein, many modifications and changes will occur to those skilled in the art. It is, therefore, to be understood that the appended claims are intended to cover all such modifications and changes as fall within the true spirit of the invention.

### ELEMENT LIST

- 10: syngas turbine
- 12: combustor
- 14: hydrocarbon fuel
- 16: oxidant
- 18: steam
- 20: synthesis gas
- 22: turbo-expander
- 24: work
- 26: quenched syngas
- 28: air compressor
- 29: shaft
- 30: inlet
- 32: outlet
- 34: recycled tail-gas
- 36: generator
- 40: shaft
- 42: electricity
- 50: GTL system
- 52: air separation unit
- 54: gas processing unit
- 56: gas synthesis unit
- 58: Fischer-Tropsch processing unit
- 60: cracking unit
- 100: GTL system
- 102: air separation unit
- 104: gas processing unit
- 106: combustor
- 108: Fischer-Tropsch processing unit
- 110: cracking unit
- 111: synthesis gas
- 112: turbo-expander
- 114: work
- 116: quenched synthesis gas
- 118: generator
- 120: shaft
- 122: electricity
- 128: air compressor
- 134: work
- 136: shaft
- 138: tail-gas
- 200: close-coupled syngas turbine
- 212: combustor
- 222: turbo-expander
- 300: close-coupled syngas turbine
- 312: can-annular combustor
- 322: turbo-expander
- 400: close-coupled syngas turbine
- 412: can-annular combustor
- 422: turbo-expander
- 450: transition piece
- 452: first end
- 454: opposite end
- 500: close-coupled syngas turbine
- 512: combustor
- 522: turbo-expander
- 550: transition piece
- 552: first end
- 554: opposite end

## Claims

1. A syngas turbine (10) comprising:
a combustor (12) for reacting a hydrocarbon fuel (14) with at least one of an oxidant (16) and steam (18) at an elevated temperature and pressure to produce a synthesis gas (20) enriched with carbon monoxide and hydrogen gas; and
a turbo-expander (22) in flow communication with said combustor for extracting work from and for quenching said synthesis gas (20).

2. A syngas turbine (10) in accordance with claim 1, wherein said turbo-expander (22) is a multi-stage axial flow turbine.

3. A syngas turbine (10) in accordance with claim 1 or claim 2, wherein said work extracted from said turbo-expander (22) is used for at least one of producing electric energy or energizing system components.

4. A gas to liquid system (100) comprising:
an air separation unit (102) for separating oxygen from air;
a gas-processing unit (104) for preparing natural gas for conversion in the combustor (106);
a combustor (106) for reacting said oxygen with said natural gas at an elevated temperature and pressure to produce a synthesis gas (111) enriched with carbon monoxide and hydrogen gas;
a turbo-expander (112) in flow communication with said combustor (106) for extracting work from and for quenching said synthesis gas (111); and
a Fischer-Tropsch processing unit (108) for receiving said quenched synthesis gas (116) and for catalytically converting said quenched synthesis (116) gas into a long-chain hydrocarbon fluid; and
a cracking unit (110) for fractionating said long-chain hydrocarbon fluid into at least one useful product.

5. A gas to liquid system (100) in accordance with claim 4, wherein said at least one useful product is synthetic diesel fuel.

6. A gas to liquid system (100) in accordance with claim 4, wherein said at least one useful product is selected from the group consisting of synthetic kerosene, ethanol, dimethyl ether, naphtha and combinations thereof.

7. A gas to liquid system (100) in accordance with any one of claims 4 to 6, wherein said work extracted from said turbo-expander (112) is utilized by said air separation unit (102).

8. A gas to liquid system (100) in accordance with any one of claims 4 to 7, further comprising a generator (118) mechanically coupled to said turbo-expander (112) for converting the work extracted from said turbo-expander (112) into electricity.

9. A method of syngas production comprising the steps of:
introducing an oxidant and a hydrocarbon fuel into a combustion chamber;
reacting said oxidant and said hydrocarbon fuel at an elevated temperature and pressure to produce a synthesis gas enriched with carbon monoxide and hydrogen gas; and
flowing said synthesis gas through a turbo-expander to extract work from and quench the synthesis gas.

10. A close-coupled syngas turbine (200) comprising:
a combustor (212) for reacting a hydrocarbon fuel with at least one of an oxidant and steam at an elevated temperature and pressure to produce a synthesis gas enriched with carbon monoxide and hydrogen gas; and
a turbo-expander (222) directly coupled to said combustor (212) for extracting work from and for quenching said synthesis gas.
